# EUROPEAN PATENT APPLICATION

(11) **EP 2 716 755 A1**
(43) Date of publication of application: **09.04.2014**
(21) Application number: 11859266.6
(22) Date of filing: 25.02.2011
(51) Int. Cl.: C12N 15/09, A01H 5/06, A01N 63/00, C07K 14/08, C12N 7/00

(54) **ATTENUATED VIRUS OF LILY MOTTLE VIRUS**

(71) Applicant: Kikkoman Corporation, Noda-shi, Chiba 278-8601 (JP); Utsunomiya University, Utsunomiya-shi, Tochigi, 321-8505 (JP); Nippon Del Monte Corporation, Chuo-ku, Tokyo 103-0016 (JP)
(72) Inventor: KOMINATO, Masayuki, Tokyo 105-0003 (JP); SAYAMA, Haruki, Tokyo 105-0003 (JP); KUMAGAI, Naoki, Utsunomiya-shi Tochigi 321-8505 (JP); NATSUAKI, Tomohide, Utsunomiya-shi Tochigi 321-8505 (JP)
(74) Representative: HOFFMANN EITLE
(86) International application number: PCT/JP2011/054370
(87) International publication number: WO 2012/114520

(57) **Abstract**

The present invention provides an attenuated lily mottle virus which does not cause any mosaic symptom in lily leaves or faint mosaic symptom, if any.

SEQUENCE LISTING

<110>  Kikkoman Corporation

<120>  Attenuated Lily Mottle Virus

<130>  K0770ABP0007PCT

<160>  7

<170>  PatentIn version 3.1

<210>  1
<211>  1374
<212>  DNA
<213>  Lily mottle virus

<400>  1
tcgggcccag gcgataaatt ctgggcagga tttaactccc aattcctcag gatcaaaccc      60

aaggaaatac cacacacttg cacatcaaac ttcaacgtct tcgagtgtgg gcaggtcgcg     120

gctcgtatgt gccaagctct gtatccatgt gggagaatta catgtcaaca gtgtgcgtct     180

gagtatctgg agctgacgaa ggttgaactc ggggaaaagc tcagtgatgg tttgtataga     240

acaagttcgc aaattcgtga aaaccaccaa gatttcgccc atgtggcaaa catactcaac     300

gtaatagctg atcttctgag cctcaagaac aacaacatgg aggcgttcac agaagtccac     360

aaactcatag gtgagcaaac acagtcacca ttcacacacc tcaatcgctt gaactcaata     420

ctgatcaaag gcagcgatat gagcagcaat gagctgtatg aatgttcaga tcgcattcgt     480

gagctagcgc gattccagaa gaacaggaca gataacatca agaaactgga cgttagcgtg     540

tttcggaata aagtatcctc caagtcatat ttcaacctgg acttgatgtg tgacaaccaa     600

cttgacaaga acggaaactt cgaatggggc cagcgaggct atcatgccaa acggttctta     660

tcgaattatt ttgaggttgt caacacacag aatgcctacc gagagcacac actgaggaag     720

catccgaatg gcatgcgaga gctcgcaatt gggaaattta tagtgtcaac aaactttgaa     780

gtgtttcgca agagcatgga agggaagcgc ataccacaaa tgcctgttac tgaggcttgc     840

ctgagtcagc gtggagagcg tttcatttac cagtgctgct gtgtaacaaa cgaagatggc     900

actccactcg agtcgacatt caaaatgcca acgaagaaca cactcgtgct tgggaacacg     960

ggtgatgaga aatttgtcga catgcccacc gacatggatg agaagatgta tatagctcga    1020

gagggctact gctacatcaa catatttttc gccatgctaa taaacgttcg tgaaagtcaa    1080

gcaaaggcat tcacgaagat ggtgcgagac cgcctcgtac ccgagcttgg aacatggcca    1140

accatgatcg atgtggcaac agcatgccac acattgactg tcttcttccc agacacactg    1200

agcgcagaac tcccgcgaat tctcgtggac cacaacacta agacgatgca tgtgattgac    1260

tcatacggat ctgttagcac tggctaccac cagttaaagg ctaacacagt gagtcagcta    1320

gtcctctttg ctagtgacac gctgaagtca gaaatgaagc actaccaggt cggc          1374

<210>  2
<211>  9645
<212>  DNA
<213>  Lily mottle virus

<400>  2
   aaaataaaac aacccaacaa gactcaatac aacacaaccc agcagctttc aaactctctt      60
   ctttgctttc aagcaagttt ctgattcctc ttttggactc acaaccagtt tcaaagcact     120
   cttctccact caacgcattc acaactgaaa gagatggcct cattcacggt cggatcgatc     180
   cttgtgaaca ccacaagcgt cctgaaagaa agctttgcac agaaagtact cacaacacct     240
   ccagtgttgg tagcacagcc tgtgcagagg agctttggtg cggcacggat gtgcgcacgc     300
   aaagcaattg cagatgatga agagaaggtg gccaaagcct tctcagctct ggaagcacgg     360
   tgtgctgcca gggtctcaag gatgggaacc atccgcgtga aggtcacgcg gtctggaacc     420
   tcgcgggcgc agccactccc aaaaagggag caggcacgtc ttaggaaggc tgaacgtaag     480
   gcacgcttgg atcgtgaaga gtttctgaaa ggaagcccca ctgtcattga tcacatcagt     540
   atagctggtg gcgcgcagcc ttcacaggac atggaggagc caatgtccac acgaaaggca     600
   ccatggtatt cagtgagcaa gaaagctcga aaagtaatcc cgcgctcccc aacactgaat     660
   gaaactgggc ttgcatcatt gatgcgagcc atcgcaaaga ttggggccaa gaattccatg     720
   aaagttgaaa tcgtggatga gcgaagagtg caagcacgct acataaagcg tgacgcaggg     780
   tcatacctgc aagtgcgcgt cgcgcaccat gagggcctaa ggcgtaggcg tgacttgaaa     840
   ctctctgcat ttgcagacca gtgcttaaca caacttgcca gcaccaccca cgggacgaag     900
   taccaccacg taacccagat taaagcgggt gacagtggga tcgttctgtt agcacgcaga     960
   ctctttgggt ggcacagcgg tagttgtagt caaatttttg tcgtgcgtgg aaatcatgaa    1020
   ggcaagttat atgaggcacg gcgcaaggtg actctaacta tgacacacaa gatgatccac    1080
   tactcgggcc caggcgataa attctgggca ggatttaact cccaattcct caggatcaaa    1140
   cccaaggaaa taccacacac ttgcacatca aacttcaacg tcttcgagtg tgggcaggtc    1200
   gcggctcgta tgtgccaagc tctgtatcca tgtgggagaa ttacatgtca acagtgtgcg    1260
   tctgagtatc tggagctgac gaaggttgaa ctcggggaaa agctcagtga tggtttgtat    1320
   agaacaagtt cgcaaattcg tgaaaaccac caagatttcg cccatgtggc aaacatactc    1380
   aacgtaatag ctgatcttct gagcctcaag aacaacaaca tggaggcgtt cacagaagtc    1440
   cacaaactca taggtgagca aacacagtca ccattcacac acctcaatcg cttgaactca    1500
   atactgatca aaggcagcga tatgagcagc aatgagctgt atgaatgttc agatcgcatt    1560
   cgtgagctag cgcgattcca gaagaacagg acagataaca tcaagaaact ggacgttagc    1620
   gtgtttcgga ataaagtatc ctccaagtca tatttcaacc tggacttgat gtgtgacaac    1680
   caacttgaca agaacggaaa cttcgaatgg ggccagcgag gctatcatgc caaacggttc 1740
   ttatcgaatt attttgaggt tgtcaacaca cagaatgcct accgagagca cacactgagg 1800
   aagcatccga atggcatgcg agagctcgca attgggaaat ttatagtgtc aacaaacttt 1860
   gaagtgtttc gcaagagcat ggaagggaag cgcataccac aaatgcctgt tactgaggct 1920
   tgcctgagtc agcgtggaga gcgtttcatt taccagtgct gctgtgtaac aaacgaagat 1980
   ggcactccac tcgagtcgac attcaaaatg ccaacgaaga acacactcgt gcttgggaac 2040
   acgggtgatg agaaatttgt cgacatgccc accgacatgg atgagaagat gtatatagct 2100
   cgagagggct actgctacat caacatattt ttcgccatgc taataaacgt tcgtgaaagt 2160
   caagcaaagg cattcacgaa gatggtgcga gaccgcctcg tacccgagct tggaacatgg 2220
   ccaaccatga tcgatgtggc aacagcatgc cacacattga ctgtcttctt cccagacaca 2280
   ctgagcgcag aactcccgcg aattctcgtg gaccacaaca ctaagacgat gcatgtgatt 2340
   gactcatacg gatctgttag cactggctac caccagttaa aggctaacac agtgagtcag 2400
   ctagtcctct ttgctagtga cacgctgaag tcagaaatga agcactacca ggtcggcggg 2460
   aagaagttca cagtcgaata cgaagcagtt aagctactaa taaaggctat atataggcct 2520
   aagcttatgc ggacactgat ggaggatgag ccctacctaa tttgtctcgc aatgtgctct 2580
   ccaggagtgc ttattgctct gttcaacagt ggatccctcg aaaccgcaat caactactgg 2640
   attgcgcggc acaaagacac tgcacaaatt tttgcgctac tcactgtact cgccacaaaa 2700
   gtgtcagtcg ctcgcacact cacagagcaa ctggcactat tgagcgatca tgcgacagat 2760
   gtgctcaaga tcatggatag aacgtttgcc acgcagcact ctgtttcact agtgcatacg 2820
   ttcttgatgc aactggagga gaagcgacac acggatgtct cactcgtggg ctcaggtttc 2880
   gccaatttgt gtgcaacaac gcatgagctt atggaaaaaa attatctggc ggagttggat 2940
   gcgtcctggc gagatctaag cttgttggaa aagtgctcac aaatgcgttg gtcatttcaa 3000
   tcaccaaagc agttttcaaa cgaattgcac cccagcgcgt caaccgattt gaacggtcgg 3060
   tacaacacat ctgtgcatgc gctttccaca agatatatga ataacctacg taagagtgta 3120
   tgtacaaagt atcataatat gaaaatacga acgcatcagt ttataggtag acaagtgtgt 3180
   aattcaataa tgggatcaag taggttcttc ccggatttag cacaatgcgt gcatatatta 3240
   gcgatattta gtataatgtt aaacattatt cgcactatta acatatatat tagtgagtac 3300
   atacgcctga agaactgcga actctcgcag attgaagaga aacgctggaa taaactactc 3360
   cacacatttg atatgatgga gaagatagat ggacagaaac ctacgctaat tgaattcact 3420
   aagcacctta aacaatggga tccagagttg tactctgagt ttcatagcat attacaggat 3480
   gaggatgagg atactgagga ggtgcaattc caggctaaac cggacgggga gagaaacctc 3540
   gaacgaatta tggctataat tgcactgact atgatggtct ttgacgctga tcgtagtgat 3600
   tgcgtgtata aggtgcttaa caagttgaaa ggtttgctga acacagtgca ccaagagccg 3660
   gtgaaatttc agagtattga tgacatccga gacattctcg aagagcgtga aatggtcatc 3720
   gactttgaga tctcagcgga cgactacaac atcaacaaac tcgctggtgc aacattcgaa 3780
   cagtggtgga gcaaccagat ggaatgcaat aatgtgctgc cacactatag aacggaaggc 3840
   ttcttcatgg agtttacacg agcgaatgca gcacatgtag cgaatgaaat agcacacggc 3900
   ccacacaagg acttgctgat ccgtggtgct gttggatctg gaaagtcgac agggctccct 3960
   ttctatttga gcaagcgcgg acgcgttttg atgcttgagc ccactaggcc attggccagt 4020
   aatgtgcaca agcaactggc aggggatccg ttcctggtca gcgcaacgct aaagatgcgg 4080
   ggagaaacca catttggctc agctccaatc acagtgatga caagtggata tgcgttccat 4140
   tactacgcca acaatcccaa ccagctacgc gactttgagt tcatcatctt tgatgagtgc 4200
   catgtcaatg acagctcagc gatggctttc cggagcttgc tcgcagagta cacgtacgac 4260
   ggacgcatca tcaaggtgtc ggcaacacca ccagggcgag aggttgagtt caccacgcag 4320
   catcctgttg aaataatacc catcgagcgg ttgacgtttc agcagttcgt tgcagtgcag 4380
   gggactggag cggtcggtga tgtgaccacg aaaggggaca atattttagt gtacgtcgct 4440
   agctacagcg aagttgataa tcttagcaaa gggttagtgg aaaagggtca caaagtcacg 4500
   aaagtggacg gtaggaccat gaaagtcgga ggtgtggaga tcatcactag cggtagccaa 4560
   gcgaggaaac actttgttgt ggccacgaat atcatcgaga acggtgtcac attggatatc 4620
   gatgttgtgg tggactttgg gttgaaagtt gtggcagagg tggacatcga ctcgcgtctc 4680
   acgcgctaca tcaagaagtc gatatcatat ggagaacgca tacagaggtt ggggcgtgtc 4740
   ggacggaata aacctggtgt cgctattcgg gttggcacca cggagaaagg attacaggca 4800
   atccctgtaa cgacggccac ggaggcagca ttcctgtgct tcgcttatgg tctaccggta 4860
   atgactaata atgtgtctac aaacatactg actaattgca cagttctgca agctcgaacg 4920
   atgatgctct ttgagatcaa cccgttctat atgtgtcatt tcgttcgatt tgatggaact 4980
   atgcacccag aggtacataa aatactaaca ccatacaagc tacgcgactc ggagataata 5040
   ctaaataaag tcgcaatacc aaataaaggt ttgatgcagt ggcccactgc gaaggaatat 5100
   gcctatcaag gatttaaaat gaatatacct gatacagtgc gactaccttt ccactcactg 5160
   gacatccctg agcggctaca tgagagaatg tggcaaatcg tcgaaacata caagggcgac 5220
   gctgggtttg gccgcataac aacagcaagc gcatgcaaga ttgcgtacac actgaagact 5280
   gatgttgcat ccattcaacg cacaatccac atattggata agttgatcga aaatgagctt 5340
   aagaagcaag agtatttccg gaacatcaca agtgcttcgt gctccagctc cagcttctca 5400
   ctcacgacga tcacaaatgc aatacgcgca cggcacatca aagatcacac tgttgaaaac 5460
   gtgagcgtcc tccaggcggc gaaggcacaa atccttgagt tcaagaacgt tacattcgac 5520
   ttggaccacg tgaatcgcat gactgagtat ggtgctttgg agtgtgtcca atttcagaca 5580
   ctggagagca tgactcaaca cctacagcta aaaggtcatt ggaataaaag tctattgacc 5640
   catgatattg tgatttgtgg ggctgtgtta ctcggttgcg tcttgatggt tgggtcctat 5700
   ttcaaggagc gatgctctgg tgtcgtgaaa cgatacaatg agaacgtgaa attccaagca 5760
   aagaacaaac gacaaagaca gcgcttgaga ttcagggagg cgcgtgacaa caagcacgcg 5820
   tacgaagttc atggtgatga tgccgacatc cagacatatt tcggatcagc ttacacgaag 5880
   aagggaaaga ctaagggtgt cacgcgaggg atgggaatca agaccaggaa atttgtaaat 5940
   atgtataatt ttgacccaac cgaatattca tttgccagat atgtcgaccc actcacaggg 6000
   tacacactag acgaacaatc tttaaccgac atagccctag tgcaggacca cttcggacgg 6060
   atcagacgga agctgatgga ggatggtgag ctcgagaagg aaagtttggc tcagaatgca 6120
   cgcttggaag tgtactttgt caagaacttg gcaagtcaaa tcctcaaaat tgacatgacg 6180
   ccacacaacc cgttgcgtgt gtgcgaccat gtcgaaacgg tggcagggtt tcccgagcgc 6240
   gatatggagc tacgccagtc tgggaaacta gtcatggttc cagctgcaga gttgcctaag 6300
   gaaaatccct atcctgaggg tgagatagtt gagtttgaaa acaagtccac attccatgga 6360
   ctgcgggatt acaatcccat agccacttgt gtgtgtaggc tcgagcacaa ctctgatgga 6420
   cacacatcga gtctatatgg tattgggtat gggtcgtata taataactaa ccaacactta 6480
   tttatacgta ataacggcac tataaacatt gagtcgcacc acggaacgta ccacatacgc 6540
   aactccacac aactacaact ttacccaatt gaggggagag acatagtgat catccagctg 6600
   ccaaaggact tcccaccatt cgcacgacgc ctgaagttcc gacatccaac aacaactgac 6660
   aaggtttgtt tggtcggcac aaactttcaa gagaaaacga caacaagcac agtgtctgaa 6720
   gctagtttga tcactcgaaa ggatgattca cacttcttta ggcactggat cagcacgaag 6780
   gatgggcaat gtggcctgcc cgcagtatca actaaggatg gctgcgtttt aggcttacac 6840
   agtttgacga gcttggtcaa tgatagtaat ttcttcattg cattccctga cgatttcgag 6900
   aagaattact tagagcgggc aaatgaattg aactgggtca agcactggaa gctgaacgtc 6960
   gacaagatct gctggggagc actgtctctc gagaaggaca aaccgagcaa catgttcaaa 7020
   ttgagcaagg acattcagcg cctggacatg gaacctgttg gactccaaag cagggaggat 7080
   aagtggttat ttgacaggtt aaatggcaat ctaaaggcta tggcacgtac tcgcaaccaa 7140
   cttgtcacaa aacatgtggt gaaggggaag tgcctacttt tcgagactta cctgaatgtt 7200
   actcctaagg cgaaagagta cttcacgcca atgatgggtg cataccaaaa gagccggcta 7260
   aacaaagaag catacattaa ggatctgttc aaatattcaa gccctatagt ggtcggtgac 7320
   gttgactgtg cagcctttga ggcagcatgt gatagcgtga ttcagctgtt cgagggtgct 7380
   ggatttggca ggtgcaatta tgttactgac gagcaggaga tcttttcggc gctgaacatg 7440
   aacgctgctg ttggggccat gtatagtgga aagaagcgtg actacttcaa ggatttcaca 7500
   gacctggaca aggaaagcat ccttcgggaa agctgcctac gcctgtacca aggcaagatg 7560
   ggagtgtgga acggctcttt gaaagcggag ttgcgagcaa aggagaaggt ggacctaaat 7620
   aaaacgcgaa cctttacagc ggcgcctgtg gatacactac ttggtgggaa aacgtgcgtg 7680
   gacgacttta acaacaggtt ttattctttg aacattgcgt gtccctggag cgttggcatg 7740
   acaaagtttt ataaaggttg ggatgaaatg ctgcgcaaat tacctgaagg atgggtttac 7800
   tgcgatgctg atggatcaca attcgacagc tcgttgtcac cctacctcat aaacgcagtt 7860
   ttgaatctac gactacactt tatggaggag tgggacgtgg gagcgctaat gctacagaac 7920
   ctgtatactg aaataatata tacaccaatt gccacacctg atggcacaat tataaagaaa 7980
   tttaaaggta ataatagtgg gcagccatca acagttgttg acaatactat tatggtcata 8040
   ttggccatac attattcata taaatatcta aagataacaa aacctctgga cgaattctgt 8100
   aagtactttg ttaatggtga tgacctattg ctggcagtgg cgcctgagtt cgaataccta 8160
   ttggaccact ttgcgaacac tttccagcaa ttaggactca actatgactt ttcgtccaga 8220
   accaaaaata gggaagaact atggttcatg tcgcaccgtg ggatcatgac ggatggcttg 8280
   tacataccga aacttgaacc ggagcgcatt gtatccatat tggaatggga cagagcggtg 8340
   gaaccagtcc acaggctgga ggcgatctgc gcatctatga ttgaggcgtg gggatacccg 8400
   cagttgttgc atgaaatcag aaagttctac tactgggtgc tcgaacaagc cccttattct 8460
   gaacttgcac aacttggtaa agctccatac ttatcggaaa ccgctcttcg tgcgctgtac 8520
   actggtgagg caacgagcca ggacgagctc gagcgttact tggcagcctt aaacttagaa 8580
   ggtgaatgtg gtggcgcaga gattgtggcg ttccaggcaa atgagacact caatgctgga 8640
   gcttccagtt ccacacaagc gagccgatcg actcgacctg aggctgcaat tgatgtggca 8700
   ccgcaacaga gttctgaggc tggagtgcgt gatcgtgatg tcgacgccgg caccgtggga 8760
   acataccaaa tcccgcgact gaaggcacta gcaacaaaga tcaacgtacc caaggtcaag 8820
   gggcgaatga tagtgaacac tgggcacctt gtgaattaca acccagacca aacagatatt 8880
   tcaaatacaa ggtcaaccca gaagcagttt gaggcttggt acaacgcagt gaaagacgag 8940
   tatggtctca acgacgagag tatggctctc acaatgaatg gtctgatggt ttggtgcata 9000
   gagaatggca cctcaccaaa tgtaaatggc gtgtggctca tgatggacgg agatcagcaa 9060
   gttgaatttc ctttacgtcc tatacttgaa cacgcaaaac cgacgctgcg ccaaattatg 9120
   gcgcatttct caaacctcgc tgaagcttat attgagaagc aaaatttaga gaaaccgtac 9180
   atgcctaggt acggccttca gcgaaatctc accgacttca atctagcacg atttgctttt 9240
   gatttctatg aagttacttc acgcacgcca gcacgcgcta aagaagcaca cttccaaatg 9300
   aaaacagccg ctttacgcgg aaaacaatcg aggctatttg ggctagatgg aaaggtgaac 9360
   acccaagatg aggacacgga gaggcataca gcagatgatg tcaataaaaa catgcactcc 9420
   ttacttggaa tttctatgta aatcctcgaa taaacagcac tagtaacttg ctaatatatc 9480
   tatctcgttt attccgtagt atcctatcta ttagctcaac tctttgtgtt atagtgtggt 9540
   ttaccatgaa cgcacattgt agttgagaac cgagctcctt gttggtgtct agttgcagag 9600
   aggcttcctc gcgtcttctt agccactagt cttggaagaa gggac 9645

<210> 3
<211> 33
<212> DNA
<213> Artificial

<220>
<223> primer

<400> 3
aaaataaaac aaaccaacaa gactcaatac aac 33

<210> 4
<211> 21
<212> DNA
<213> Artificial

<220>
<223> primer

<400> 4
gtggcgagta cagtgagaag c 21

<210> 5
<211> 35
<212> DNA
<213> Artificial

<220>
<223> primer

<400> 5
aactgcggcc gcatgtcggg cccaggcgat aaatt 35

<210> 6
<211> 35
<212> DNA
<213> Artificial

<220>
<223> primer

<400> 6
tctagcggcc gcctagccga cctggtagtg cttca 35

<210>  7
<211>  458
<212>  PRT
<213>  Lily mottle virus

<400>  7

Ser Gly Pro Gly Asp Lys Phe Trp Ala Gly Phe Asn Ser Gln Phe Leu
1               5                   10                  15

Arg Ile Lys Pro Lys Glu Ile Pro His Thr Cys Thr Ser Asn Phe Asn
            20                  25                  30

Val Phe Glu Cys Gly Gln Val Ala Ala Arg Met Cys Gln Ala Leu Tyr
        35                  40                  45

Pro Cys Gly Arg Ile Thr Cys Gln Gln Cys Ala Ser Glu Tyr Leu Glu
    50                  55                  60

Leu Thr Lys Val Glu Leu Gly Glu Lys Leu Ser Asp Gly Leu Tyr Arg
65                  70                  75                  80

Thr Ser Ser Gln Ile Arg Glu Asn His Gln Asp Phe Ala His Val Ala
                85                  90                  95

Asn Ile Leu Asn Val Ile Ala Asp Leu Leu Ser Leu Lys Asn Asn Asn
            100                 105                 110

Met Glu Ala Phe Thr Glu Val His Lys Leu Ile Gly Glu Gln Thr Gln
        115                 120                 125

Ser Pro Phe Thr His Leu Asn Arg Leu Asn Ser Ile Leu Ile Lys Gly
    130                 135                 140

Ser Asp Met Ser Ser Asn Glu Leu Tyr Glu Cys Ser Asp Arg Ile Arg
145                 150                 155                 160

Glu Leu Ala Arg Phe Gln Lys Asn Arg Thr Asp Asn Ile Lys Lys Leu
                165                 170                 175

Asp Val Ser Val Phe Arg Asn Lys Val Ser Ser Lys Ser Tyr Phe Asn
            180                 185                 190

Leu Asp Leu Met Cys Asp Asn Gln Leu Asp Lys Asn Gly Asn Phe Glu
        195                 200                 205

Trp Gly Gln Arg Gly Tyr His Ala Lys Arg Phe Leu Ser Asn Tyr Phe
    210                 215                 220

Glu Val Val Asn Thr Gln Asn Ala Tyr Arg Glu His Thr Leu Arg Lys
225                 230                 235                 240

His Pro Asn Gly Met Arg Glu Leu Ala Ile Gly Lys Phe Ile Val Ser
                245                 250                 255

Thr Asn Phe Glu Val Phe Arg Lys Ser Met Glu Gly Lys Arg Ile Pro
            260                 265                 270

Gln Met Pro Val Thr Glu Ala Cys Leu Ser Gln Arg Gly Glu Arg Phe
        275                 280                 285

Ile Tyr Gln Cys Cys Cys Val Thr Asn Glu Asp Gly Thr Pro Leu Glu
    290                 295                 300

Ser Thr Phe Lys Met Pro Thr Lys Asn Thr Leu Val Leu Gly Asn Thr
305                 310                 315                 320

Gly Asp Glu Lys Phe Val Asp Met Pro Thr Asp Met Asp Glu Lys Met
                325                 330                 335

Tyr Ile Ala Arg Glu Gly Tyr Cys Tyr Ile Asn Ile Phe Phe Ala Met
            340                 345                 350

Leu Ile Asn Val Arg Glu Ser Gln Ala Lys Ala Phe Thr Lys Met Val
        355                 360                 365

Arg Asp Arg Leu Val Pro Glu Leu Gly Thr Trp Pro Thr Met Ile Asp
    370                 375                 380

Val Ala Thr Ala Cys His Thr Leu Thr Val Phe Phe Pro Asp Thr Leu
385                 390                 395                 400

Ser Ala Glu Leu Pro Arg Ile Leu Val Asp His Asn Thr Lys Thr Met
                405                 410                 415

His Val Ile Asp Ser Tyr Gly Ser Val Ser Thr Gly Tyr His Gln Leu
            420                 425                 430

Lys Ala Asn Thr Val Ser Gln Leu Val Leu Phe Ala Ser Asp Thr Leu
        435                 440                 445

Lys Ser Glu Met Lys His Tyr Gln Val Gly
    450                 455

## Description

### Technical Field

The present invention relates to an attenuated lily mottle virus that infects lilies to cause disease damage.

### Background Art

Lily mosaic disease is a viral disease that occurs in lilies. Its causative virus is reportedly lily mottle virus (hereinafter occasionally referred to as "LMoV"). The LMoV virus is classified into the genus *Potyvirus* of the family Potyviridae and known to have a single-stranded RNA genome.

Since lilies are vegetatively propagated crops, lilies infected by LMoV transmit LMoV along with their vegetative propagation and thus result in great damage by infection. Thus, the infection of LMoV in lilies leads to marked decrease in revenues from cut flower production and bulb production. There are no agricultural chemicals effective for eliminating LMoV in lily, as in other plant viruses.

To protect lilies from LMoV, virus-free lilies have been produced by meristem culture. However, viral infection via aphids occurs in farm fields in a short time. The infection of LMoV is generally prevented by spraying insecticides against such vector aphids or by use of repellent materials or blocking materials. The effects of these approaches, however, are insufficient.

Reportedly, use of attenuated viruses is a current effective approach for plant viral diseases. Accordingly, attenuated viruses of causative viruses for plant viral diseases are under development around the world.

### Summary of Invention

### Technical Problem

An object to be solved by the present invention is to provide an attenuated lily mottle virus, the virus causing a lily to have no or faint mosaic symptoms, if any when inoculated to the lily.

### Solution to Problem

The present inventors have conducted diligent studies to solve the object and consequently completed the present invention by isolating attenuated strains of LMoV from the field and subsequent screening that enabled controlling lily mosaic disease caused by LMoV.

Specifically, the present invention is as follows:
[1] An attenuated lily mottle virus which does not cause any mosaic symptom in lily leaves or faint mosaic symptom, if any.
[2] The attenuated lily mottle virus according to [1], comprising an RNA represented by the nucleotide sequence of SEQ ID NO: 1 (provided that T in the sequence is replaced with U).
[3] The attenuated lily mottle virus according to [1] or [2], comprising any of the following peptides:
   (1) a peptide represented by the amino acid sequence of SEQ ID NO: 7; and
   (2) a peptide represented by an amino acid sequence derived from the amino acid sequence of SEQ ID NO: 7 by the deletion, addition, and/or substitution of one or several amino acid(s).
[4] A nucleic acid comprising the nucleotide sequence of SEQ ID NO: 1 or a nucleotide sequence complementary to the nucleotide sequence of SEQ ID NO: 1 (provided that T in the nucleotide sequence is replaced with U when the nucleic acid is an RNA).
[5] A method for controlling lily mottle virus disease, comprising a step of:
   inoculating the attenuated lily mottle virus according to any of [1] to [3] to a lily.
[6] A method for preparing a lily mottle virus-resistant plant, comprising a step of:
   inoculating the attenuated lily mottle virus according to any of [1] to [3] to a lily.
[7] A method for propagating a lily mottle virus-resistant plant, comprising a step of:
   propagating, by scale propagation or tissue culture, a lily infected by the attenuated lily mottle virus according to any of [1] to [3].
[8] A lily mottle virus-resistant plant obtained by the inoculation of the attenuated lily mottle virus according to any of [1] to [3].
[9] A lily plant tissue infected by the attenuated lily mottle virus according to any of [1] to [3].
[10] The lily plant tissue according to [9], wherein the tissue is a scale or a bulb.

### Advantageous Effects of Invention

The present invention can provide an attenuated lily mottle virus which protects lilies effectively and efficiently from lily mosaic disease.

### Brief Description of Drawings

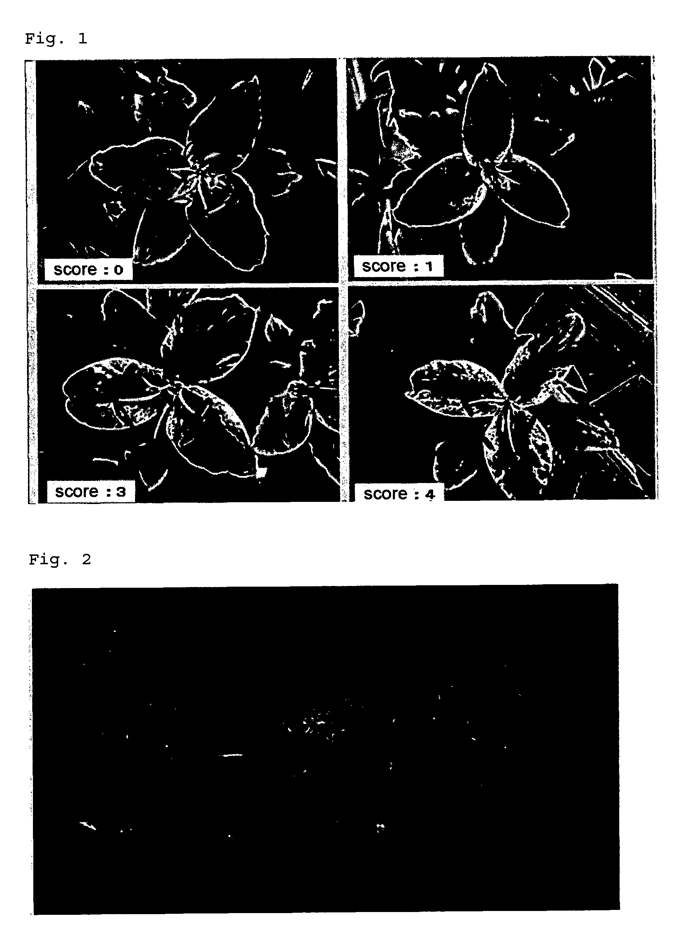

[Figure 1] The virus symptom of a lily cultivar "Newton" with pink flower color inoculated with the virus is evaluated on a 5-point scale: no symptom: 0, faint: 1, mild: 2, faintly severe: 3, and severe: 4. Figure 1 shows the petals of the cultivar "Newton" with pink flower color evaluated as a score of no symptom: 0, faint: 1, faintly severe: 3, or severe: 4.
[Figure 2] Figure 2 shows a symptom in a test plant *Nicotiana benthamiana* inoculated with a PVX vector containing the Hc-Pro region of NDM-LM1 strain, an attenuated virus, or a virulent strain. Results about the test plant inoculated with only a "PVX vector" are shown on the left. Results about the test plant inoculated with the "PVX vector containing the Hc-Pro region of the NDM-LM1 strain" are shown in the middle. Results about the test plant inoculated with the "PVX vector containing the Hc-Pro region of the virulent strain" are shown on the right.

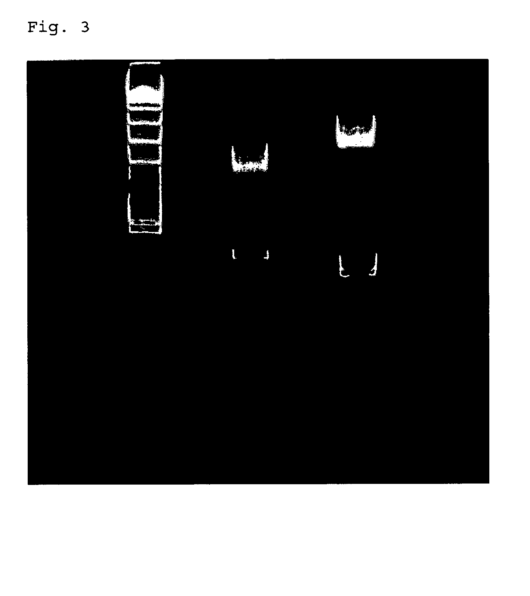

[Figure 3] Figure 3 shows the results of electrophoresis of a restriction enzyme SphI-cleaved RT-PCR amplification product of the HC-Pro gene from a plant with inoculated with a PVX vector containing the HC-Pro gene. Size markers are shown on the left. The SphI-cleaved products of the HC-Pro gene derived from the attenuated strain are shown in the middle. The SphI-cleaved products of the HC-Pro gene derived from the virulent strain are shown on the right.

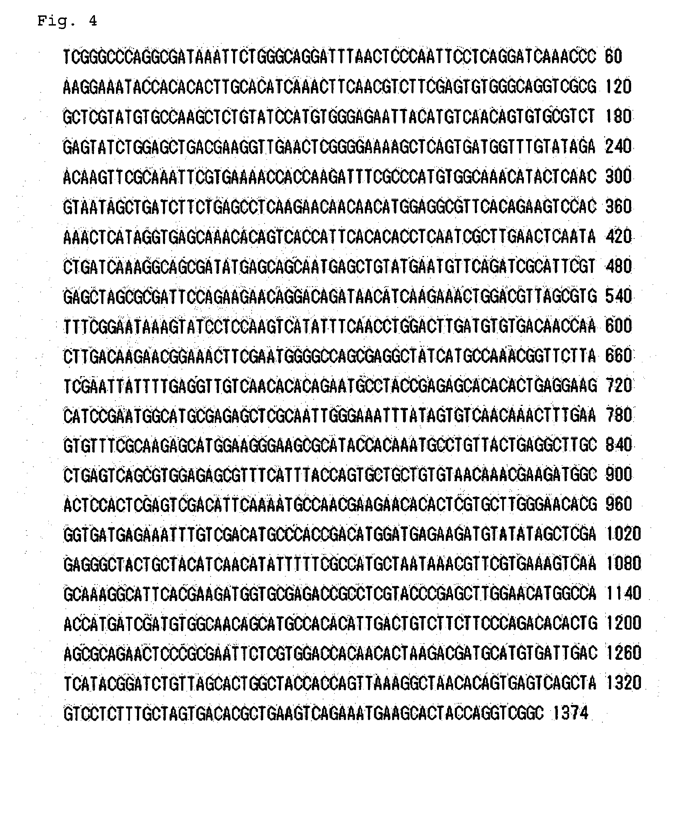

[Figure 4] Figure 4 shows the nucleotide sequence of SEQ ID NO: 1.

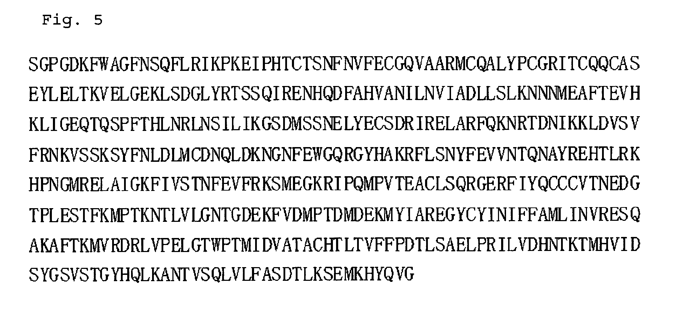

[Figure 5] Figure 5 shows the amino acid sequence of SEQ ID NO: 7.

### Description of Embodiments

Hereinafter, each embodiment for carrying out the present invention will be described in detail. However, the present invention is not limited by the embodiments shown below, and various changes or modifications can be made therein without departing from the gist of the present invention.

### (1) Attenuated lily mottle virus

The attenuated lily mottle virus (hereinafter occasionally referred to as an "attenuated virus") in the present invention is an attenuated virus which does not cause any mosaic symptom in lily leaves or faint mosaic symptom, if any. Preferably, the attenuated virus in the present invention further does not cause any variegated symptom in flowers or faint variegated symptom, if any.

One example of the attenuated virus in the present invention includes an NDM-LM1 strain. The NDM-LM1 strain is an attenuated virus obtained as shown below.

Attenuated LMoV strains are obtained from a symptom-free lily cultivar with pink flower color by tissue blot analysis to select LMoV12-1-1 strain. The LMoV12-1-1 strain is further screened to reduce the adverse effects observed in the cultivar with pink flower color. For example, an attenuated LMoV, LMoV12-1-9 (NDM-LM1 strain) which has an exceedingly little adverse effect on sensitive cultivars with pink flower color, is screened from LMoV12-1-1 strain by selecting repeatedly lily plants that have no mosaic symptom on their petals over 9 generations.

The attenuated virus in the present invention, preferably the NDM-LM1 strain which is the attenuated virus in the present invention, has an RNA represented by the nucleotide sequence described in SEQ ID NO: 1 (provided that T in the sequence is replaced with U).

The attenuated virus in the present invention having the RNA represented by the nucleotide sequence described in SEQ ID NO: 1 (provided that T in the sequence is replaced with U) can be provided as an attenuated virus which does not cause any mosaic symptom in lily leaves or faint mosaic symptom, if any. Use of this attenuated virus provides an effective and efficient method for controlling lily mosaic disease. The attenuated virus in the present invention can further save costs such as costs of agricultural chemicals, materials, fertilizers, or seedlings and reduce labor required for crop management. As a result, it provides LMoV-resistant materials with no symptom or faint symptom, if any by means of vegetative propagations such as bulb or scale propagation. Thus, the attenuated virus in the present invention is practical.

The present invention provides even a nucleic acid comprising the nucleotide sequence of SEQ ID NO: 1, the nucleic acid being isolated from the attenuated virus. The nucleotide sequence of the nucleic acid may be a nucleotide sequence having the deletion, addition, and/or substitution of one or several base(s) without departing from the object of the present invention and is preferably an RNA having the nucleotide sequence described in SEQ ID NO: 1 provided that T in SEQ ID NO: 1 is replaced with U when the nucleic acid is an RNA. Alternatively, the nucleotide sequence described in SEQ ID NO: 1 may contain bases equivalent to A, T, G, C, and U, in addition to A, T, G, C, and U. The equivalent bases contained therein may be bases having substituted or modified base moieties of A, T, G, C, and U. In addition, a sugar moiety in the nucleic acid may be substituted or modified if the sugar moiety is equivalent to ribose or deoxyribose.

The present invention also provides a nucleic acid comprising a nucleotide sequence complementary to the nucleotide sequence of SEQ ID NO: 1. The nucleotide sequence of the nucleic acid, which is complementary to the nucleotide sequence of SEQ ID NO: 1, may be a nucleotide sequence having the deletion, addition, and/or substitution of one or several base(s) without departing from the object of the present invention and is preferably a DNA having a nucleotide sequence complementary to the nucleotide sequence described in SEQ ID NO: 1.

The nucleotide sequence described in SEQ ID NO: 1 comprises a sequence presumably essential for the attenuated virus which does not cause any mosaic symptom in lily leaves or faint mosaic symptom, if any and corresponds to a helper component proteinase (HC-Pro) region.

The attenuated virus in the present invention, preferably the NDM-LM1 strain which is the attenuated virus in the present invention, comprises a peptide represented by the amino acid sequence described in SEQ ID NO: 7 or a peptide represented by an amino acid sequence derived from the amino acid sequence described in SEQ ID NO: 7 by the deletion, addition, and/or substitution of one or several base(s). The peptide represented by the amino acid sequence described in SEQ ID NO: 7 or the peptide represented by an amino acid sequence derived from the amino acid sequence described in SEQ ID NO: 7 by the deletion, addition, and/or substitution of one or several base(s) is a peptide encoded by the nucleic acid comprising the nucleotide sequence of SEQ ID NO: 1 or a nucleotide sequence derived from the nucleotide sequence of SEQ ID NO: 1 by the deletion, addition, and/or substitution of one or several base(s). Also, the peptide represented by the amino acid sequence described in SEQ ID NO: 7 or the peptide represented by an amino acid sequence derived from the amino acid sequence described in SEQ ID NO: 7 by the deletion, addition, and/or substitution of one or several base(s) corresponds to a helper component proteinase (HC-Pro) region.

The attenuated virus in the present invention comprising the peptide represented by the sequence can be provided as an attenuated virus which does not cause any mosaic symptom in lily leaves or faint mosaic symptom, if any. Use of this attenuated virus provides an effective and efficient method for controlling lily mosaic disease. The attenuated virus in the present invention can further save costs such as costs of agricultural chemicals, materials, fertilizers, or seedlings and reduce labor required for crop management. As a result, it provides LMoV-resistant materials with no symptom or faint symptom, if any by means of vegetative propagations such as bulb or scale propagation. Thus, the attenuated virus in the present invention is practical.

The attenuated virus in the present invention is not particularly limited to the NDM-LM1 strain and includes a wide range of viruses having the property that does not cause any mosaic symptom in lily leaves or faint mosaic symptom, if any. Preferably, the attenuated virus in the present invention further exhibits the property that does not cause any variegated symptom in flowers or faint variegated symptom, if any. The attenuated virus in the present invention is not particularly limited as long as the virus does not cause any mosaic symptom in lily leaves or faint mosaic symptom, if any. The attenuated virus in the present invention is preferably a virus which does not cause any mosaic symptom in lily leaves or no mosaic symptom, if any and does not cause any variegated symptom in flowers or faint variegated symptom, if any. Alternatively, the attenuated virus in the present invention may be a virus which does not cause any variegated symptom in flowers or faint variegated symptom, if any.

### (2) Method for controlling lily mottle virus disease

The method for controlling lily mottle virus disease in the present invention involves inoculating the attenuated lily mottle virus to a lily to thereby controlling the lily mottle virus disease.

The attenuated virus can be inoculated in advance to a lily to thereby prevent the lily from being infected by LMoV.

The term "lily mottle virus disease" refers to the condition of an LMoV-infected lily having a mosaic symptom in its leaf and/or a variegated symptom in its flower due to infection. This disease may further cause an LMoV-infected lily dwarfing and/or reduce the bulb size.

The lily to be inoculated with the attenuated virus is not particularly limited as long as the lily belongs to the genus *Lilium.* Examples thereof include Oriental hybrids, Asiatic hybrids, and Easter lily.

The inoculation timing of the attenuated virus is not particularly limited. Preferably, the attenuated virus is inoculated at a seedling stage.

The inoculation of the attenuated virus to the lily can be carried out, for example, by rubbing an attenuated virus-infected leaf as a source of inoculum and inoculating the inoculums to lily seedlings (height: approximately 15 cm) using a swab coated with celite as an abrasive. The infected leaf can be a proximal leaf of a flower located at the highest position in a plant that has been confirmed to have no mosaic symptom in its leaves after growing the inoculated seedlings till blooming all flowers. The infected leaf is preferably a proximal leaf of a flower located at the highest position in a plant that has been confirmed to have no mosaic symptom in its leaves and no variegated symptom in its flowers.

Each lily seedling inoculated with the attenuated virus can be cultivated to thereby prepare an adult lily inoculated with the attenuated virus. For example, bulbs or scales resistant to LMoV (resistant source) can be obtained from the adult lily plants infected with the attenuated LMoV. Examples of the lily plant tissue include, but not particularly limited to, leaves, stems, roots, flowers, cut flowers, bulbs, scales, cell masses, calluses, and protoplasts obtained from lily plants.

### (3) Method for preparing lily mottle virus-resistant plant

The method for preparing a lily mottle virus-resistant plant in the present invention involves inoculating the attenuated lily mottle virus in the present invention to a lily to thereby prepare a lily mottle virus-resistant plant that exhibits resistance to lily mottle virus by virtue of viral functions exerted by the attenuated virus.

The attenuated virus can be inoculated in advance to a lily to thereby prepare a lily that exhibits resistance to infection of LMoV.

The cultivars of the lily, the inoculation method, and the timing of inoculation can be the same as in the protective method described above.

### (4) Method for propagating lily mottle virus-resistant plant

The method for propagating a lily mottle virus-resistant plant in the present invention involves propagating, by bulb propagation, scale propagation, or tissue culture, a lily infected by the attenuated lily mottle virus in the present invention to thereby propagate a lily mottle virus-resistant plant so that a next-generation lily is prepared.

Bulbs infected by the attenuated virus are planted and cultivated in a farm field or a facility. Lily mottle virus-resistant plants can be propagated to thereby obtain new plant tissues such as bulbs and scales.

These lily mottle-resistant plants or lily plant tissues obtained by the propagating the lily mottle virus-resistant plants have resistance to virulent lily mottle virus by virtue of the attenuated virus that has already been delivered into their tissues. Likewise, individuals allowed to proliferate by the tissue culture of a lily infected by the attenuated virus also have resistance to virulent lily mottle virus.

Since the effect of the attenuated virus can be sustained by vegetative propagation, a producer does not actually need to inoculate the attenuated virus in every cultivation and can continuously obtain the protective effect of the attenuated virus over seasons by harvesting bulbs or scales through usual cultivation.

### Examples

Hereinafter, the present invention will be described more specifically with reference to Examples. However, the scope of the present invention is not limited by only these Examples.

### [Isolation of attenuated virus NDM-LM1 strain]

Attenuated LMoV strains were screened by tissue blot analysis from virus symptom-free plants (600 plants) at a bud stage of a lily cultivar "Sorbonne" with pink flower color that was sensitive to the adverse effects (which mean a variegated symptom in flowers and a mosaic symptom in leaves) of the virus. The obtained strain was designated as LMoV12-1-1 strain. The LMoV12-1-1 strain was inoculated to a lily cultivar "Newton" with pink flower color to investigate a virus symptom. In this procedure, the virus symptom that appeared in petals was evaluated on a 5-point scale: no symptom: 0, faint: 1, mild: 2, faintly severe: 3, and severe: 4.

Figure 1 shows the petals of the cultivar "Newton" with pink flower color evaluated as a score of no symptom: 0, faint: 1, faintly severe: 3, or severe: 4.

As a result of the evaluation, the first-generation lilies inoculated with the LMoV12-1-1 strain had a score of 1.14, whereas uninoculated lilies had a score of 0.17. Lilies inoculated with a virulent LMoV strain GLS4 had a score of 4.00, which exhibited the most severe degree of the virus symptom (n = 36).

The virulent LMoV strain GLS4 (hereinafter occasionally referred to as a "virulent GLS4 strain") used was a virulent virus obtained from a lily that exhibited a severe mosaic symptom observed in the farm field of lily bulb production based on scale propagation in Niigata prefecture, Japan.

Since the lilies inoculated with the LMoV12-1-1 strain had a score higher than that of the uninoculated lilies and were confirmed to have a variegated symptom in their flowers, the virus symptom appearing in petals was investigated in order to reduce the adverse effects of the LMoV12-1-1 strain in the cultivar with pink flower color. As a result, a more severe virus symptom was observed in late blooming flowers located at an upper position than the earliest blooming flowers located at a lower position. On the basis of this finding, the LMoV12-1-1 strain was screened from the LMoV12-1-1 inoculated plants which did not show any virus symptom in upper leaves over 9 generations. As a result, the score 1.14 of the first-generation lilies inoculated with the LMoV12-1-1 strain was able to be reduced to 0.16 in the ninth-generation lilies infected by an LMoV12-1-9 strain (NDM-LM1 strain) (n = 25). These ninth-generation lilies were evaluated as being marketable as cut flowers without problems. Table 1 shows the results about the severity (score) of a virus symptom in the petals of the first-generation to ninth-generation lilies of the cultivar "Newton" with pink flower color.

**[Table 1]**

| Table 1. Reduction in the severity (score) of a virus symptom in the petals of the cultivar with pink flower color | |
|---|---|
| The number of generations | Score |
| 1st generation | 1.14 |
| 2nd generation | 0.38 |
| 3rd generation | 0.89 |
| 4th generation | 0.86 |
| 5th generation | 0.78 |
| 6th generation | 0.80 |
| 7th generation | 0.45 |
| 8th generation | 0.40 |
| 9th generation | 0.16 |

| | |
|---|---|
| Score of virus symptom in petal: no symptom: 0, faint: 1, mild: 2, faintly severe: 3, and severe: 4 | |

A full-length DNA sequence homologous to the full-length RNA sequence of the attenuated virus LMoV12-1-9 strain (NDM-LM1 strain) obtained from the ninth-generation lily was inserted to a plasmid. This plasmid, which was designated as pNDMLM1, was accepted by National Institute of Technology and Evaluation, Patent Microorganisms Depositary on Feb. 16, 2011 and deposited under Deposition No. FERM-ABP11343. The NDM-LM1 strain is being preserved by the present applicant, and transfer prescribed by Rule 27-3 of the Japanese Patent Law Enforcement Regulation is guaranteed by the present applicant.

The nucleotide sequence inserted to the deposited plasmid is shown in SEQ ID NO: 2.

### [Characteristics of attenuated virus NDM-LM1 strain]

An aphid transmissibility test was conducted on lilies infected by the NDM-LM1 strain or the virulent GLS4 strain.

Each fasted aphid (alate) was transferred to the leaves of a lily cultivar "Aktiva" infected by the virulent GLS4 strain or the attenuated virus NDM-LM1 strain, followed by acquisition feeding. Then, the fed aphid was transferred to virus-uninfected Takasago lily leaves, followed by inoculation feeding for 1 day on the Takasago lilies (5 aphids/plant). Ten plants of Takasago lily in each of the virulent GLS4 strain inoculation feeding group and the NDM-LM1 strain inoculation feeding group were used in the test.

The presence or absence of detectable viruses was assayed by tissue blot analysis (antigen-antibody reaction) to determine the rate of transmission of each virus to the Takasago lily via the aphid.

As a result, use of *Aphis gossypii* Glover (alate) as the aphid produced 80% rate of transmission of the virulent GLS4 strain, but produced 0% rate of transmission of the NDM-LM1 strain.

As a result of conducting a transmissibility test using *Aulacorthum solani* in the same way as above, the rate of transmission of the virulent GLS4 strain was 70%, whereas the rate of transmission of the NDM-LM1 strain was 0%. As a result of conducting a transmissibility test using *Myzus persicae,* the rate of transmission of the virulent GLS4 strain was 50%, whereas the rate of transmission of the NDM-LM1 strain was 0%.

These results showed that 3 types of aphids (*Aphis gossypii* Glover, *Aulacorthum solani,* and *Myzus persicae)* transmit the virulent GLS4 strain, but do not transmit the NDM-LM1 strain. The results of the aphid transmissibility test are shown in Table 2.

[Table 2]

**Table 2. Aphid transmissibility test**

| Aphid | Virulent GLS4 strain | NDM-LM1 strain |
|---|---|---|
| *Aphis gossypii* Glover | 80% | 0% |
| *Aulacorthum solani* | 70% | 0% |
| *Myzus persicae* | 50% | 0% |

### [Protective effect of attenuated virus NDM-LM1 strain against virulent GLS4 strain]

As a control test, an uninoculated group (group inoculated with neither the NDM-LM1 strain nor the virulent GLS4 strain), an NDM-LM1 strain group (group inoculated with the NDM-LM1 strain but with no virulent GLS4 strain), and a virulent GLS4 strain group (group inoculated with no NDM-LM1 strain but with the virulent GLS4 strain) were investigated (n = 12) in the same way as above.

The results are shown in Table 3.

[Table 3]

**Table 3. Protective effect of the attenuated LMoV strain, NDM-LM1 against the virulent GLS4 strain in a lily**

| Group | Height (cm) | The number of flower buds per plant | The number of blooming flowers per plant |
|---|---|---|---|
| Uninoculated | 77.5 b | 3.2 | 1.0 b |
| NDM-LM1 strain | 79.3 b | 3.1 | 2.3 c |
| Virulent GLS4 strain | 63.3 a | 3.0 | 0.3 a |
| NDM-LM1 strain + Virulent GLS4 strain | 73.9 b | 3.1 | 2.4 c |

| | | | |
|---|---|---|---|
| Different characters in the same column represent a significant difference (p < 0.05). | | | |

The results of Table 3 demonstrated that the lilies inoculated with the virulent GLS4 strain (virulent GLS4 group) tended to be dwarfed and delayed blooming stage, whereas the lilies inoculated with the virulent GLS4 strain after infection of the attenuated NDM-LM1 (NDM-LM1 group + virulent GLS4 strain group) had a height comparable to that of the uninoculated lilies (uninoculated group) and had an earlier blooming stage. This showed the NDM-LM1 strain has a remarkable protective effect against the virulent strain of LMoV.

Also, the inoculation of the NDM-LM1 strain was shown to result in the height of the plant comparable to that of the uninoculated plant and slightly hasten its blooming stage without influencing the number of flowers per plant. This demonstrated that the inoculation of the NDM-LM1 strain hastens the blooming stage and also showed that the inoculation of the attenuated virus rarely produced adverse effects in lilies. Thus, the attenuated virus NDM-LM1 strain was shown to be usable as a virus vaccine against LMoV.

### [Farm field cultivation test on attenuated virus NDM-LM1 strain]

The NDM-LM1 strain was inoculated to a lily cultivar "Casablanca". A practical application test was conducted in four farm fields in order to compare an inoculated zone with an uninoculated zone. In one farm field (A), a virus symptom was observed in 5 out of 29 lily plants in the uninoculated zone. LMoV was detected in 3 out of the 5 plants, while cucumber mosaic virus (CMV) and LMoV were detected in the remaining 2 plants. In this farm field, 27 lily plants in the NDM-LM1 strain-inoculated zone exhibited no virus symptom. The results are shown in Table 4.

[Table 4]

**Table 4. Results of the farm field test on the lilies infected by the attenuated virus NDM-LM1 strain**

| Investigation item | Zone | Farm field | | | |
|---|---|---|---|---|---|
| | | A | B | C | D |
| The number of plants having virus symptom^{a} | Uninoculated | 5/29 | 0/29 | 0/28 | 0/24 |
| | NDM-LM1 strain-inoculated | 0/27* | 0/25 | 0/23 | 0/28 |
| Height (cm) | Uninoculated | 39.0 | 50.5 | 48.5 | 78.1 |
| | NDM-LM1 strain-inoculated | 40.3 | 50.4 | 50.7 | 78.0 |
| The number of flower buds per plant | Uninoculated | 1.2 | 2.7 | 1.8 | 1.3 |
| | NDM-LM1 strain-inoculated | 1.9 | 2.7 | 2.0 | 1.9 |
| Yield of bulb per plant (g) | Uninoculated | 141.2 | 165.3 | 161.9 | 141.5 |
| | NDM-LM1 strain-inoculated | 150.4* | 161.9 | 163.0 | 141.2 |

| | | | | | |
|---|---|---|---|---|---|
| ^{a} : The number of plants having virus symptom/ the number of tested plants * : Significantly different from uninoculated group (p<0.05) | | | | | |

The results of Table 4 showed that the NDM-LM1 strain has a protective effect against viral infection in the farm field infected with LMoV and CMV.

Bulbs were harvested from this farm field, and their weights were compared between the inoculated zone and the uninoculated zone. As a result, the bulbs harvested from the NDM-LM1 strain-inoculated zone were significantly higher in weight than those from the uninoculated zone. This demonstrated that the NDM-LM1 strain also has a protective effect on bulb growth in the farm field affected by LMoV and CMV. In the other farm fields (B, C, and D), no virus symptom was observed, and the height of each lily and the weight of each bulb derived from the NDM-LM1 strain-inoculated zone were comparable to those from the uninoculated zone, demonstrating few adverse effects. The number of flower buds tended to be slightly larger in the NDM-LM1 strain-inoculated zone than the uninoculated zone.

### [Determination of nucleotide sequence]

The whole nucleotide sequences of the virulent strain GLS4 strain and the attenuated virus NDM-LM1 strain were determined.

In order to determine the whole nucleotide sequence of LMoV, primers shown below were designed, and RT-PCR products were obtained. The RT-PCR products were purified and then inserted by TA cloning to plasmid vectors, with which E. *coli* was then transformed. Plasmid DNA was purified from the transformed E. *coli* DH5α and sequenced using BigDye Terminator v3.1 Cycle Sequencing Kit and ABI PRISM3100 Genetic Analyzer.
LMoV_IC_2F: AAAATAAAACAAACCAACAAGACTCAATACAAC (SEQ ID NO: 3)
LMoV_IC_2R: GTGGCGAGTACAGTGAGAAGC (SEQ ID NO: 4)

The attenuated virus NDM-LM1 strain had the nucleotide sequence represented by SEQ ID NO: 2. The nucleotide sequence represented by SEQ ID NO: 2 consists of 5' UTR, P1, Hc-Pro, P3, 6K1, CI, 6K2, VPg, a functionally unidentified region, NIa, NIb, CP, 3' UTR, and PIPO.

The LMoV helper component proteinase (HC-Pro) regions of the virulent strain GLS4 strain and the attenuated strain NDM-LM1 strain were separately inserted to PVX vectors to prepare infectious chimeric clones. The presence or absence of change in plant pathogenicity by each inserted HC-Pro region was investigated.

Foreign gene expression PVX vectors (pgR106) developed by Baulcombe et al. were used. The HC-Pro gene of the virulent strain or the attenuated strain was inserted to a foreign gene insertion site (Takken, F.L., Luderer, R., Gabriels, S.H., Westerink, N., Lu, R., de Wit, P.J., and Joosten, M.H. (2000). A functional cloning strategy, based on a binary PVX-expression vector, to isolate HR-inducing cDNAs of plant pathogens. Plant J., vol. 24, p. 275-283).

An F primer and an R primer shown in Table 5 were used for this insertion of the HC-Pro gene to the PVX vector (pgR106). The F primer and the R primer each have a restriction enzyme site NotI. The F primer and the R primer can be used in common to both the virulent GLS4 strain and the attenuated virus NDM-LM1 strain. The HC-Pro region was amplified by PCR using these primers. The obtained PCR products were treated with NotI and inserted to the NotI sites of the PVX vectors (pgR106). After the insertion, the nucleotide sequence of the HC-Pro gene was confirmed to be successfully inserted.

[Table 5]

**Table 5. Sequences of primers for inserting the HC-Pro gene to the PVX vector (pgR106)**

| |
|---|
| • F primer (4 bases + NotI + start codon + first 20 bases of HC-Pro): |
| AACTGCGGCCGCATGTCGGGCCCAGGCGATAAATT Tm: 66.5 (SEQ ID NO: 5) |
| • R primer (strand complementary to last 20 bases of HC-Pro + stop codon + NotI + 4 bases): |
| TCTAGCGGCCGCCTAGCCGACCTGGTAGTGCTTCA Tm: 67.7 (SEQ ID NO: 6) |

The HC-Pro gene-inserted plasmid DNAs (infections chimeric clones) were purified in large amounts and inoculated to test plants *Nicotiana benthamiana* by the particle gun method. Change in the pathogenicity of potato virus X (PVX) by the inserted HC-Pro gene was evaluated. The particle gun manufactured by Bio-Rad Laboratories, Inc. was used, and the inoculation conditions followed the manual of the product.

The virulent strain-derived HC-Pro gene inoculated to the test plant considerably strengthened the pathogenicity of PVX, whereas the test plant inoculated with the attenuated strain-derived HC-Pro gene was not influenced by the pathogenicity of PVX (Figure 2).

The HC-Pro region was amplified by RT-PCR from each plant inoculated with the PVX vector containing each HC-Pro gene and cleaved by a restriction enzyme SphI, followed by electrophoresis. The virulent strain-derived HC-Pro gene was confirmed in the plant having severe symptoms, while the attenuated strain-derived HC-Pro gene was confirmed in the plant having no symptom. The results of electrophoresis are shown in Figure 3. In Figure 3, size markers are shown on the left. The SphI-cleaved products of the HC-Pro gene derived from the attenuated strain are shown in the middle. The SphI-cleaved products of the HC-Pro gene derived from the virulent strain are shown on the right. The patterns of SphI cleavage differed between the attenuated strain and the virulent strain. The patterns of SphI cleavage were not changed even after exhibition of a symptom by the inoculated plants (data not shown), demonstrating that the inoculation of the attenuated strain-derived Hc-Pro gene causes the plant to have no symptom, whereas the inoculation of the virulent strain-derived Hc-Pro gene causes the plant to exhibit severe symptoms. Since the pattern of SphI cleavage in the attenuated strain - derived Hc-Pro gene had two SphI cleavage sites, a band of approximately 430 bases, which was not observed in the pattern of SphI cleavage in the virulent strain-derived Hc-Pro gene, was confirmed.

### Industrial Applicability

The attenuated lily mottle virus of the present invention can serve as effective control means for lily mottle virus disease. The present invention therefore brings about ripple effects such as the high-quality and stable production of cut flowers and bulbs of lilies, price stabilization based on such production, decrease in labor and cost required for controls against viral diseases, the supply of safe and secure cut flowers and bulbs resulting from reduced use of agricultural chemicals, and the promotion of environmentally friendly agriculture. Accordingly, the present invention is practical and makes a great contribution to the society.

### Sequence Listing Free Text

SEQ ID NO: 1 represents the nucleotide sequence of the Hc-Pro region of an NDM-LM1 strain.
SEQ ID NO: 2 represents a nucleotide sequence that corresponds to the full-length sequence of the NDM-LM1 strain and is inserted in a plasmid carried by deposited *E. coli.*
SEQ ID NO: 3 represents the nucleotide sequence of an F primer used for determining the whole nucleotide sequences of a virulent strain GLS4 and the attenuated virus NDM-LM1 strain.
SEQ ID NO: 4 represents the nucleotide sequence of an R primer used for determining the whole nucleotide sequences of a virulent strain GLS4 and the attenuated virus NDM-LM1 strain.
SEQ ID NO: 5 represents the nucleotide sequence of an F primer used for inserting the HC-Pro gene to PVX (pgR106).
SEQ ID NO: 6 represents the nucleotide sequence of an R primer used for inserting the HC-Pro gene to PVX (pgR106).
SEQ ID NO: 7 represents the amino acid sequence of the Hc-Pro region of the NDM-LM1 strain.

## Claims

1. An attenuated lily mottle virus which does not cause any mosaic symptom in lily leaves or faint mosaic symptom, if any.

2. The attenuated lily mottle virus according to claim 1, comprising an RNA represented by the nucleotide sequence of SEQ ID NO: 1 (provided that T in the sequence is replaced with U).

3. The attenuated lily mottle virus according to claim 1 or 2, comprising any of the following peptides:
(1) a peptide represented by the amino acid sequence of SEQ ID NO: 7; and
(2) a peptide represented by an amino acid sequence derived from the amino acid sequence of SEQ ID NO: 7 by the deletion, addition, and/or substitution of one or several amino acid(s).

4. A nucleic acid comprising the nucleotide sequence of SEQ ID NO: 1 or a nucleotide sequence complementary to the nucleotide sequence of SEQ ID NO: 1 (provided that T in the nucleotide sequence is replaced with U when the nucleic acid is an RNA).

5. A method for controlling lily mottle virus disease, comprising a step of:
inoculating the attenuated lily mottle virus according to any one of claims 1 to 3 to a lily.

6. A method for preparing a lily mottle virus-resistant plant, comprising a step of:
inoculating the attenuated lily mottle virus according to any one of claims 1 to 3 to a lily.

7. A method for propagating a lily mottle virus-resistant plant, comprising a step of:
propagating, by scale propagation or tissue culture, a lily infected by the attenuated lily mottle virus according to any one of claims 1 to 3.
[Cla

8. A lily mottle virus-resistant plant obtained by the inoculation of the attenuated lily mottle virus according to any one of claims 1 to 3.

9. A lily plant tissue infected by the attenuated lily mottle virus according to any one of claims 1 to 3.

10. The lily plant tissue according to claim 9, wherein the tissue is a scale or a bulb.
